(19) Europäisches Patentamt
European Patent Office
Office européen des brevets

(11) Veröffentlichungsnummer: **0 225 290**
**B1**

# (12) EUROPÄISCHE PATENTSCHRIFT

(45) Veröffentlichungstag der Patentschrift: **23.11.89**

(51) Int. Cl.⁴: **A01N 43/20**, C07D 303/32

(21) Anmeldenummer: **86810546.1**

(22) Anmeldetag: **26.11.86**

## (54) Herbizid wirkende Epoxide.

(30) Priorität: **02.12.85 CH 5131/85**
**30.06.86 CH 2617/86**

(43) Veröffentlichungstag der Anmeldung: **10.06.87 Patentblatt 87/24**

(45) Bekanntmachung des Hinweises auf die Patenterteilung: **23.11.89 Patentblatt 89/47**

(84) Benannte Vertragsstaaten: **BE CH DE FR GB IT LI NL**

(56) Entgegenhaltungen:
**EP-A- 0 114 567**
**EP-A- 0 117 578**
**EP-A- 0 138 085**
**DE-A- 2 520 165**

(73) Patentinhaber: **CIBA-GEIGY AG, Klybeckstrasse 141, CH-4002 Basel(CH)**

(72) Erfinder: **Rempfler, Hermann, Dr., Brücklismattstrasse 16, CH-4107 Ettingen(CH)**
Erfinder: **Kunz, Walter, Dr., Buchenstrasse 9, CH-4104 Oberwil(CH)**

**EP 0 225 290 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein herbizides und den Pflanzenwuchs regulierendes Mittel, welches als Wirkstoff Epoxide enthält, sowie die Verwendung dieses Mittels oder der Epoxide zur selektiven Unkrautbekämpfung in Nutzplanzenkulturen oder zur Regulierung, insbesondere Hemmung des Pflanzenwachstums.

Bei den Wirkstoffen handelt es sich um 2-Benzoyl-2-phenyl-oxirane entsprechend der Formel I

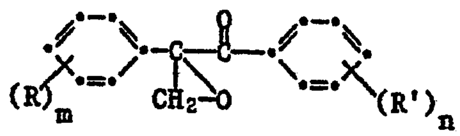

(I)

worin m und n unabhängig voneinander je Null, eins, zwei oder drei R und R′ unabhängig voneinander je ein Halogenatom, eine $C_1$-$C_4$-Alkyl, $C_1$-$C_4$-Alkoxy, $C_1$-$C_4$-Halogenalkyl-, $C_1$-$C_4$-Halogenalkoxy- oder die Nitro- oder Cyangruppe oder einen durch $(R)_m$ substituierten Phenoxyrest bedeutet, sowie um deren optisch aktive Enantiomere.

Die als Wirkstoffe verwendeten 2-Benzoyl-2-phenyl-oxirane und ihre Herstellung sind z.T. bekannt und in Chem. Ber 91 2710(1953) sowie in den Europäischen Patentanmeldungen EP-A 114 567 und 117 578 beschrieben. Sie dienten dort als Zwischenprodukte zur Herstellung von fungiziden Wirkstoffen.

Die Herstellung der Oxirane der Formel I geschieht in an sich bekannter Weise durch Epoxidierung aus den zugrundeliegenden Styrolderivaten der Formel II

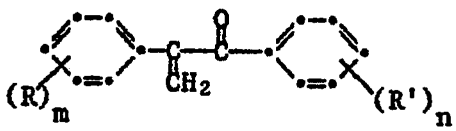

(II)

worin m, n, R und R′ die unter Formel I gegebene Bedeutung haben.

Die Oxidierung erfolgt beispielsweise mittels Persäuren, wie Peressigsäure, tert.Butylhydroperoxid, m-Chlorperbenzoesäure, $H_2O_2$ usw. gegebenenfalls in Gegenwart von Basen wie NaOH, KOH, $NaHCO_3$ in gängigen reaktionsinerten Lösungsmitteln. Hierbei können Carbonylkomplexe von Uebergangsmetallen, speziell z.B. $MO(CO)_6$ als Katalysator eingesetzt werden.

Die Styrolderivate der Formel II können aus den entsprechenden Ketonen der Formel III

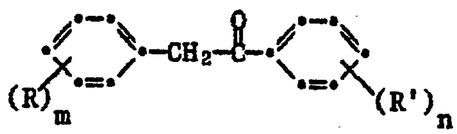

(III)

worin m, n, R und R′ die unter der Formel I gegebene Bedeutung haben, gemäss Ang. Chemie 1976 261 durch Reaktion mit einem Dimethylmethylenammoniumhalogenid erhalten werden (Siehe dazu auch Heterocycles 12 (1979)938).

Das Reaktionsschema kann wie folgt dargestellt werden:

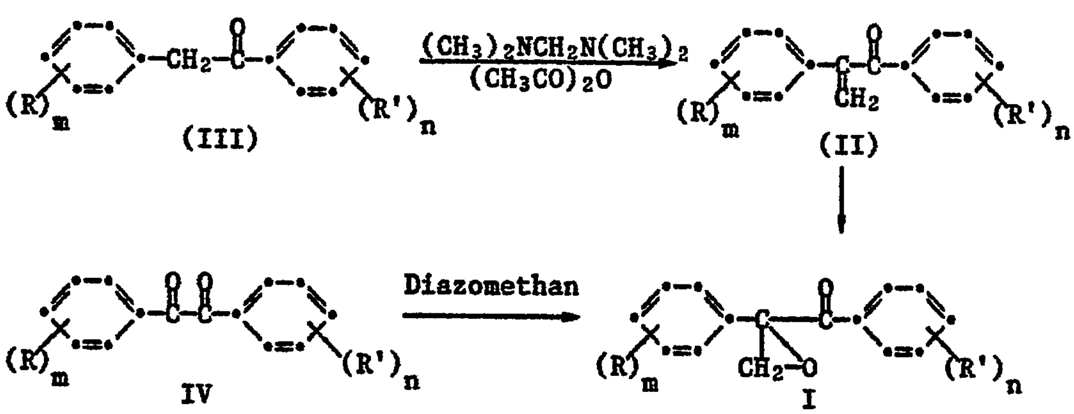


(III) (II) IV I

Die Oxirane der Formel I lassen sich auch herstellen, indem man ein Diketon (Dibenzoyl) der Formel IV

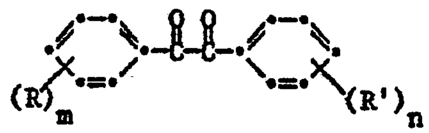

(IV)

worin m, n, R und R′ die unter der Formel I gegebene Bedeutung haben, mit Diazomethan in Gegenwart eines Lösungsmittels und NaOH umsetzt. (Siehe dazu Chem. Ber. 91 (1958)2710 oder J. Am. Chem. Soc. 87 (1965)1353).

Die erfindungsgemässen Oxirane haben ein chirales Kohlenstoffatom im Molekül und liegen als Racemat vor

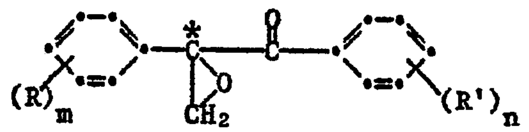

(I*)

Sie können aber relativ einfach durch Chromatographie über Acetylcellulose in ihre enantiomeren Formen getrennt werden. Die Enantiomeren verhalten sich verschieden in ihrer Wirkung und sind ebenfalls Gegenstand dieser Erfindung.

Einige Oxirane sind als Zwischenprodukte für die Herstellung von fungiziden Wirkstoffen bekannt geworden, andere sind neu. Neu sind die optisch aktiven Enantiomere sowie die Verbindungen der Formel Ia

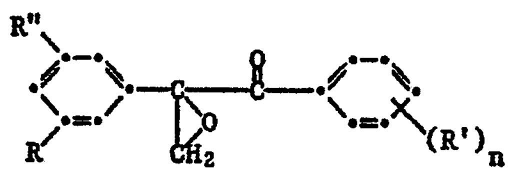

(Ia)

worin R Halogen, $C_1$-$C_4$-Alkyl oder $C_1$-$C_4$-Alkoxy, R″ Wasserstoff oder dasselbe wie R bedeuten und R′ und n die unter der Formel I gegebene Bedeutung haben als Racemat oder optisch aktives Enantiomeres.

Besonders aktiv sind die Verbindungen, in denen R″ Wasserstoff ist.

Bei der Herstellung der genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindung wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone, wie Aceton, Diethylketon, Methylethylketon und Gemische solcher Lösungsmittel untereinander.

Einige der Oxirane der Formel I waren als Zwischenprodukte für die Herstellung fungizider Wirkstoffe bekannt. Überraschenderweise hat es sich nun gezeigt, daß diese Verbindungen herbizide und den Pflanzenwuchs regulierende, vor allem hemmende, Eigenwirkungen haben. Sie eignen sich als selektive Herbizide in Kulturen von Nutzpflanzen, vor allem im Reis. Ihre Wirkung ist besonders gegen monokotyle Pflanzen, d.h. Schadgräser ausgeprägt während dikotyle Kulturen sowie auch Getreidearten geschont werden.

Besonders aktiv haben sich diejenigen Mittel gezeigt, welche ein 2-Benzoyl-2-phenyl-oxiran der Formel I enthalten, in dem m und n unabhängig voneinander je Null, eins, zwei oder drei und R und R′ unabhängig voneinander je Fluor, Chlor oder Brom, Methyl, Methoxy oder Trifluormethyl bedeuten oder R auch ein durch $(R)_m$ substituierter Phenoxyrest ist.

Weiterhin aktiv sind Mittel die einen 2-Benzoyl-2-phenyl-oxiran-Wirkstoff der Formel I enthalten, worin m und n unabhängig voneinander je Null, eins, zwei oder drei und R und R′ unabhängig voneinander je Fluor, Chlor, Methyl, Methoxy oder Trifluormethyl bedeutet.

Besonders aufgefallen sind folgende Wirkstoffe:

2-Benzoyl-2-phenyl-oxiran,
2-(4-Brombenzoyl-2-phenyl)-oxiran,
2-(4-Chlorbenzoyl)-2-(4-chlorphenyl)oxiran,
2-(4-Chlorbenzoyl)-2-(2-chlorphenyl)-oxiran,
2-Benzoyl-2-p-tolyl-oxiran,
2-(2-Chlorbenzoyl)-2-phenyl-oxiran,

2-Anisoyl-2-phenyl-oxiran,
2-(4-Chlorbenzoyl)-2(3-bromphenyl)-oxiran sowie
2-(4-Chlorbenzoyl)-2-phenyl-oxiran.

Die Verbindungen der Formel I besitzen herbizide und das Pflanzenwachstum regulierende, insbesondere hemmende Wirkung. Sie sind besonders aktiv gegen monokotyle Pflanzen d.h. Gräser.

Ein grosser Vorteil ist dabei, dass die neuen Verbindungen der Formel I sich gegenüber dikotylen Kulturpflanzen aber auch monokotylen, wie Getreide, Mais oder Reis, selektiv verhalten und zur Unkrautbekämpfung in solchen Kulturen eingesetzt werden können.

Die Wirkstoffe der Formel I werden in der Regel mit Aufwandmengen von 0,005 bis 4 kg/ha insbesondere 0,01 bis 1 kg/ha erfolgreich eingesetzt.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzplanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer order organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents und Emulsifiers Annual" MC Publishing Corp., Ridgewood, New Jersey, 1979.

Dr. Helmut Stache "Tensid Taschenbuch" Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

| | |
|---|---|
| <u>Emulgierbare Konzentrate:</u> | |
| Wirkstoff der Formel I | 1 bis 20% bevorzugt 5 bis 10% |
| oberflächenaktives Mittel | 5 bis 30%, vorzugsweise 10 bis 20% |
| flüssiges Trägermittel | 50 bis 94%, vorzugsweise 70 bis 85%. |
| <u>Stäube:</u> | |
| Wirkstoff der Formel I | 0,1 bis 10%, vorzugsweise 0,1 bis 1% |
| festes Trägermittel | 99,9 bis 90%, vorzugsweise 99,9 bis 99%. |
| <u>Suspensions-Konzentrate:</u> | |
| Wirkstoff der Formel I | 5 bis 75%, vorzugsweise 10 bis 50% |
| Wasser | 94 bis 25%, vorzugsweise 90 bis 30% |
| oberflächenaktives Mittel | 1 bis 40%, vorzugsweise 2 bis 30% |
| <u>Benetzbare Pulver:</u> | |
| Wirkstoff der Formel I | 0,5 bis 90%, vorzugsweise 1 bis 80% |
| oberflächenaktives Mittel | 0,5 bis 20%, vorzugsweise 1 bis 15% |
| festes Trägermittel | 5 bis 95%, vorzugsweise 15 bis 90% |
| <u>Granulate:</u> | |
| Wirkstoff der Formel I | 0,5 bis 30%, vorzugsweise 3 bis 15% |
| festes Trägermittel | 99,5 bis 70%, vorzugsweise 97 bis 85% |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe zu beschränken. Temperaturen sind in Celsiusgraden angegeben. Prozente und Teile beziehen sich auf das Gewicht.

<u>Beispiel 1: Herstellung von 1-Benzoyl-1-(2,4-dichlorphenyl)-oxiran</u>

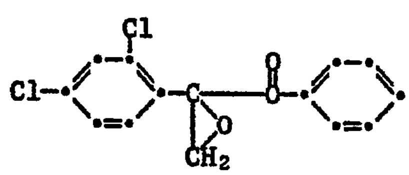

Zu 16,5 g 2-(2,4-Dichlorphenyl)-3-phenyl-prop-1-en-3-on in 225 ml Methanol werden unter Rühren 22,2 ml Wasserstoffperoxid 30%ig und dann innert 0,75 Stunden bei max. +3°C unter Kühlen 12,4 ml 3N Natronlauge zugetropft. Nach weiterem Rühren während 5 Stunden wird mit Eiswasser versetzt, mit Methylenchlorid extrahiert, die Extrakte mit Wasser und Natriumhydrogensulfit gewaschen (Peroxid-Test) und im Vakuum eingedampft. Nach dem Trocknen verblieben 7,3 g rohes Epoxid, das durch Destillation am Hochvakuum gereinigt wird.

<u>Beispiel 2: Herstellung von 2-(2,4-Dichlorbenzoyl)-2-(2,4-dichlorphenyl)-oxiran</u>

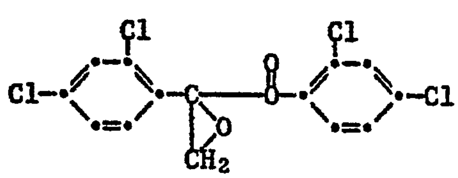

Zu einer Lösung von 3,4 g 2,2',4,4'-Tetrachlorbenzil in 50 ml Dioxan tropft man langsam unter Kühlen und Rühren eine ätherische Diazomethanlösung, welche aus 3,1 g Nitrosomethylharnstoff und wässriger Natronlauge in Aether hergestellt wurde. Nachdem alles zugetropft ist, wird 16 Stunden bei Raumtemperatur weitergerührt und die Reaktionsmischung dann zur Trockne eingedampft. Das zurückbleibende Oel ist rohes 2-(2,4-Dichlorbenzoyl)-2-(2,4-dichlorphenyl)-oxiran, welches durch Destillieren am Hochvakuum gereinigt wird.

Beispiel 3: Herstellung von 2-(4-Chlorbenzoyl)-2-(4-chlorphenyl)-oxiran

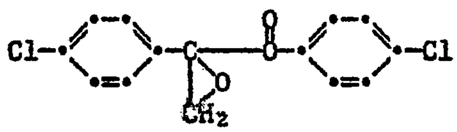

Zu einer Lösung von 5 g 4,4'-Dichlorbenzil in 50 ml Tetrahydrofuran tropft man unter Rühren und Kühlen eine ätherische Diazomethanlösung, welche durch Reaktion von 5,34 g N-Nitrosomethylharnstoff und wässrigem Natriumhydroxyd in Aether hergestellt wurde. Nachdem alles zugetropft ist, rührt man 16 Stunden bei Raumtemperatur weiter und dampft dann die Reaktionslösung ein. Das zurückbleibende Oel ist rohes 2-(4-Chlorbenzoyl)-2-(4-chlorphenyl)oxiran, welches durch Destillieren am Hochvakuum gereinigt wird.

Beispiel 4: Herstellung von 2-(4-Chlorbenzoyl)-2-(4-chlorphenyl)-oxiran

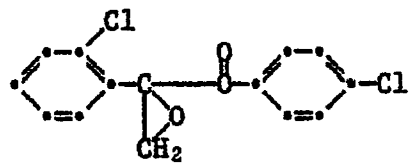

Zu einer Lösung von 150 mg 1-(4-Chlorbenzoyl)-2'-chlorstyrol in 8 ml Methanol gibt man 1 ml 10%iges Natriumhydroxyd und 1 ml 30%iges Wasserstoffperoxid. Das Reaktionsgemisch wird dann 14 Stunden bei Raumtemperatur stehen gelassen und schließlich im Vakuum eingeengt. Der Rückstand wird in Methylenchlorid aufgenommen, gewaschen, über Natriumsulfat getrocknet und im Vakuum eingeengt. Der Rückstand wird über eine Silicagelkolonne mit Methylenchlorid als Laufmittel gereinigt. Das Eluat wird eingeengt und der Rückstand mit Petroläther trituriert. Man erhält so 25 mg kristallines 2-(4-Chlorbenzoyl)-2-(2-chlorphenyl)oxiran, welches bei 65–66° schmilzt.

Das als Ausgangsmaterial benötigte 1-(4-Chlorbenzoyl)-2-chlorstyrol wird wie folgt hergestellt:
Zu 500 mg 4-Chlor-2-(2-chlorphenyl)-acetophenon gibt man 1 ml Essigsäureanhydrid und 1 ml Tetramethyldiaminoäthan und rührt die Lösung während 30 Minuten bei Raumtemperatur. Das Reaktionsgemisch wird dann eingeengt, der Rückstand in etwas Methanol aufgenommen und nochmals eingeengt. Der Rückstand wird in Petroläther trituriert. Man erhält so 280 mg 1-(4-Chlorbenzoyl)-2-chlorstyrol, welches einen Schmelzpunkt von 120–122° hat.

In analoger Weise zu diesen Beispielen werden die in der Tabelle 1 aufgeführten Verbindungen hergestellt.

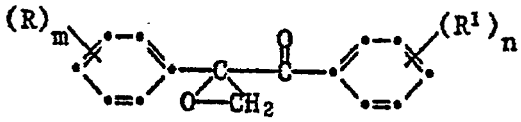

Tabelle 1

| Nr. | $(R)_m$ | $(R^1)_n$ | |
|---|---|---|---|
| 1.001 | H | H | Oel |
| 1.002 | 4-Cl | $4-OCH_3$ | Oel |
| 1.003 | 4-Cl | $4-CH_3$ | $n_D^{30}$ 1.5870 |
| 1.004 | 4-Cl | $4-CF_3$ | Oel |
| 1.005 | 4-Cl | 4-Br | $n_D^{25}$ 1.6132 |
| 1.006 | 4-Cl | H | Sdp. 150-155°/0.02 mbar $n_D^{25}$ 1.5885 |
| 1.007 | 4-Cl | 2-Cl | Oel |
| 1.008 | 4-Cl | 2-Cl | Oel |
| 1.009 | 4-Cl | 3-Cl | |
| 1.010 | 4-Cl | 4-Cl | $n_D^{30}$ 1.5994 Beispiel 3 |
| 1.011 | 4-Cl | $3,4\ Cl_2$ | $n_D^{30}$ 1.6120 |
| 1.012 | 4-Cl | $2-CH_3$ | $n_D^{25}$ 1.5840 |
| 1.013 | 4-Cl | 2-F | Sdp. 150°/0.003 mbar $n_D^{25}$ 1.5875 |
| 1.014 | 4-Br | H | $n_D^{32}$ 1.6062 |
| 1.015 | 4-Br | 4-Cl | $n_D^{30}$ 1.6110 |
| 1.016 | 4-F | H | Oel |
| 1.017 | 4-F | 4-Cl | Oel |
| 1.018 | $4-CH_3$ | H | $n_D^{20}$ 1.5792 |
| 1.019 | $4-CH_3$ | 4-Cl | $n_D^{25}$ 1.5880 |
| 1.020 | $4-CH_3$ | $4-CH_3$ | $n_D^{26}$ 1.5750 |

| Nr. | $(R)_m$ | $(R^1)_n$ | |
|---|---|---|---|
| 1.021 | 4-$OCH_3$ | H | $n_D^{26}$ 1.5943 |
| 1.022 | 4-$OCH_3$ | 2-Cl | $n_D^{30}$ 1.5745 |
| 1.023 | 2,4 $Cl_2$ | 2-Cl | $n_D^{25}$ 1.5770 |
| 1.024 | 2,4 $Cl_2$ | 2-$CH_3$ | $n_D^{25}$ 1.5790 |
| 1.025 | 2,6 $Cl_2$ | 2-Cl | Smp. 114-115° |
| 1.026 | 3,4 $Cl_2$ | 2-Cl | $n_D^{24}$ 1.5900 |
| 1.027 | 2-Cl | H | $n_D^{31}$ 1.5801 |
| 1.028 | 2-Cl | 4-Cl | Smp. 65-66° Beispiel 4 |
| 1.029 | 3-Cl | H | $n_D^{25}$ 1.5960 |
| 1.030 | 3-Cl | 4-Cl | Smp. 79-81° |
| 1.032 | 3-Cl | 4-$CH_3$ | $n_D^{25}$ 1.5860 |
| 1.033 | 3-$CF_3$ | 4-Cl | $n_D^{25}$ 1.5367 |
| 1.034 | 3-Cl | 2-$OCH_3$ 3,6 $Cl_2$ | $n_D^{25}$ 1.5772 |
| 1.035 | 3,5 $Cl_2$ | 4-Cl | $n_D^{25}$ 1.6055 |
| 1.036 | 3-$CH_3$ | H | $n_D^{30}$ 1.5810 |
| 1.037 | 3-$CH_3$ | 4-$CH_3$ | $n_D^{30}$ 1.5770 |
| 1.038 | 3-$CH_3$ | 4-Cl | Smp. 60-62° |
| 1.039 | 2-Cl | 2,4 $Cl_2$ | $n_D^{24}$ 1.5824 |
| 1.040 | H | 2-Cl | $n_D^{30}$ 1.5823 |
| 1.041 | H | 3-Cl | Oel |
| 1.042 | H | 4-Cl | $n_D^{30}$ 1.5757 |
| 1.043 | H | 4-$OCH_3$ | Smp. 76-78° |
| 1.044 | 2,4 $Cl_2$ | H | Oel Beispiel 1 |
| 1.045 | 2,4 $Cl_2$ | 2,4 $(CH_3)_2$ | Oel |
| 1.046 | 2,4 $Cl_2$ | 4-$CH_3$ | Oel |
| 1.047 | 2,4 $Cl_2$ | 2,4 $Cl_2$ | Oel Beispiel 2 |
| 1.048 | 2,4 $Cl_2$ | 4-Cl | $n_D^{22}$ 1.6045 |
| 1.049 | 4-$CH_3$ | 3-Cl | $n_D^{26}$ 1.5800 |
| 1.050 | 4-$CH_3$ | 3-$CH_3$ | $n_D^{26}$ 1.5630 |
| 1.051 | 4-$CH_3$ | 2-Cl | $n_D^{25}$ 1.5845 |
| 1.052 | 4-$CH(CH_3)_2$ | H | |

| Nr. | $(R)_m$ | $(R^1)_n$ | |
|---|---|---|---|
| 1.053 | 4-CN | 4-Cl | |
| 1.054 | 3-Cl | 2,6 $Cl_2$ | $n_D^{25}$ 1.5660 |
| 1.055 | 3-Cl | 3-$CH_3$ | |
| 1.056 | 3-Cl | 4-$NO_2$ | |
| 1.057 | 3-Cl | 2-Cl | $n_D^{25}$ 1.5930 |
| 1.058 | 3-Br | 4-Cl | $n_D^{25}$ 1.6170 |
| 1.059 | 2-$CH_3$ | H | $n_D^{25}$ 1.5838 |
| 1.060 | 2-$OCH_3$ | H | $n_D^{25}$ 1.5803 |
| 1.061 | 2-$OCH_3$ | 4-Cl | $n_D^{25}$ 1.5835 |
| 1.062 | 3,4$(OCH_3)_2$ | 4-Cl | |
| 1.063 | 3,4$(OCH_3)_2$ | H | |
| 1.064 | 3-Cl, 5-$CH_3$ | 4-Cl | $n_D^{25}$ 1.5923 |
| 1.065 | 4-(2-Chlor-4-trifluorme-thyl-phenoxy) | 4-Cl | |
| 1.066 | 4-(3-Triflu-ormethylphe-noxy) | 4-Cl | |
| 1.067 | 3-(2-Chlor-4-trifluormethyl-phenoxy) | 4-Cl | |
| 1.068 | 4(2,4-Di-chlorphe-noxy) | 4-Cl | |
| 1.069 | 2-Chlor-4-(2-Chlor-4-trifluormethyl-phenoxy) | 2-Cl | $n_D^{35}$ 1.5616 |
| 1.070 | 3-(4-Trifluormethyl-phenoxy) | H | Smp. 96-97° |
| 1.071 | 4-(2-Chlor-4-tri-fluormethyl-phenoxy) | H | $n_D^{30}$ 1.5658 |
| 1.072 | 4-(3-trifluormethyl-phenoxy) | H | $n_D^{35}$ 1.5608 |
| 1.073 | H | 2,4-$Cl_2$ | $n_D^{30}$ 1.5905 |
| 1.074 | 3-$CH_3$ | 2,3-$(CH_3)_2$ | Smp. 60-63 |
| 1.075 | 3-$CH_3$ | 2-Cl | $n_D^{25}$ 1.5825 |

| Nr. | $(R)_m$ | $(R')_n$ | |
|---|---|---|---|
| 1.076 | 3-$CH_3$ | 4-F | $n_D^{25}$ 1.5657 |
| 1.077 | 3-$CH_3$ | 2-$CH_3$ | $n_D^{30}$ 1.5615 |
| 1.078 | 3-Cl | 2,3-$(CH_3)_2$ | $n_D^{25}$ 1.5832 |
| 1.079 | 3-Cl | 2-F | $n_D^{30}$ 1.5805 |
| 1.080 | 3-Cl | 4-F | $n_D^{30}$ 1.5780 |
| 1.081 | 3-F | 4-Cl | Smp. 59-60° |
| 1.082 | 3-F | H | $n_D^{24}$ 1.5675 |
| 1.083 | 3-$OCH_3$ | 4-Cl | Smp. 71° |
| 1.084 | 3-$OCH_3$ | H | $n_D^{25}$ 1.5833 |
| 1.085 | 3-$OC_3H_7$(n) | H | $n_D^{25}$ 1.5660 |
| 1.086 | 3-$OC_3H_7$(n) | 4-Cl | $n_D^{25}$ 1.5724 |
| 1.087 | 2-Br, 5-$OC_3H_7$(n) | H | $n_D^{27}$ 1.5838 |
| 1.088 | 4-$SO_2CH_3$ | 4-Cl | Smp. 124-26° |
| 1.089 | 2,4-$Cl_2$ | 2-F | $n_D^{30}$ 1.5791 |
| 1.090 | 2-F, 6-Cl | 2-Cl | Smp. 102-104 |
| 1.091 | 2,4-$Cl_2$ | 2-$CF_3$ | $n_D^{30}$ 1.5239 |
| 1.092 | 2,4-$Cl_2$ | 2-$OCH_3$ | |
| 1.093 | 2,6-$Cl_2$ | H | Smp. 66-68° |
| 1.094 | 2-F, 6-Cl | 2-F | |
| 1.095 | 3-$CF_3$ | H | $n_D^{30}$ 1.5260 |
| 1.096 | 2-Cl, 4-$OCH_3$ | 4-Cl | $n_D^{30}$ 1.5875 |
| 1.097 | 2-Cl, 4-$OCH_3$ | 2-$CH_3$ | $n_D^{30}$ 1.5687 |
| 1.098 | 2-Cl, 4-$OCH_3$ | 2-Cl | $n_D^{30}$ 1.5759 |
| 1.099 | 3-Cl | 4-CN | |
| 1.100 | 3,5-$Cl_2$ | H | |
| 1.101 | 3-Cl | 4-Cl | (R)-Enantiomeres $[\alpha]_D$= + 196,6° (1 % in $CHCl_3$) |

| Nr. | $(R)_m$ | $(R')_n$ | |
|---|---|---|---|
| 1.102 | 3-Cl | 4-Cl | (S)-Enantiomeres $[\alpha]_D = -157°$ (1 % in $CHCl_3$) |
| 1.103 | $3-CH_3$ | 2-F | |
| 1.104 | $3-OCH_3$ | 2-F | |

Formulierungsbeispiele:

Beispiel 5:
Formulierungsbeispiele für Wirkstoffe der Formel I
(% = Gewichtsprozent)

| a) Spritzpulver | a) | b) | c) |
|---|---|---|---|
| Wirkstoff gemäss Tabelle 1 | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 | 10% | 1% |
| Octylphenolpolyäthylenglykoläther (4–5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff geemäss Tabelle I | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

| d) | Extruder Granulat | a) | b) |
|---|---|---|---|
| | Wirkstoff gemäss Tabelle I | 10% | 1% |
| | Na-Ligninsulfonat | 2% | 2% |
| | Carboxymethylcellulose | 1% | 1% |
| | Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

| e) | Umhüllungs-Granulat | |
|---|---|---|
| | Wirkstoff gemäss Tabelle I | 3% |
| | Polyäthylenglykol (MG 200) | 3% |
| | Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) | Suspensions-Konzentrat | a) | b) |
|---|---|---|---|
| | Wirkstoff gemäss Tabelle 1 | 40% | 5% |
| | Aethylenglykol | 10% | 10% |
| | Nonylphenolpolyäthylenglykoläther (15 Mol AeO) | 6% | 1% |
| | Na-Ligninsulfonat | 10% | 5% |
| | Carboxymethylcellulose | 1% | 1% |
| | 37%ige wässrige Formaldehyd-Lösung | 0,2% | 0,2% |
| | Silikonöl in Form einer 75%igen wässrigen Emulsion | 0,8% | 0,8% |
| | Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) | Salzlösung | |
|---|---|---|
| | Wirkstoff gemäss Tabelle 1 | 5% |
| | Isopropylamin | 1% |
| | Octylphenolpolyäthylenglykoläther (78 Mol AeO) | 3% |
| | Wasser | 91% |

Biologische Beispiele:

Beispiel 6: Pre-emergente Herbizid-Wirkung

Selektive Herbizidwirkung im Vorauflaufverfahren mit Getreide und Mais

Im Gewächshaus werden die Samen von Gerste, Weizen, Mais sowie von 10 Unkräutern in mit steriler Erde gefüllte Blumentöpfe (oberer Durchmesser 11 cm) gesät. Unmittelbar nach der Einsaat der Versuchspflanzen wird die Erdoberfläche mit einer wässrigen Dispersion der Wirkstoffe, erhalten aus einem 10%igen Emulsionskonzentrat behandelt. Es werden Konzentrationen von 4, 2 und 1 kg Wirksubstanz pro Hektar angewendet. Die Töpfe werden dann im Gewächshaus bei 22-25°C und 50-70 % relativer Luftfeuchtigkeit gehalten und regelmässig bewässert. Der Versuch wird nach 3 Wochen ausgewertet und der Zustand der Pflanzen nach einer 9-stufigen Notenskala bewertet.

1 = Pflanzen haben nicht gekeimt oder sind abgestorben,
2,3 = sehr starke Herbizidwirkung,
4-6 = mittlere Wirkung,
7,8 = schwache Wirkung,
9 = keine Wirkung, die Pflanze gedeiht wie unbehandelte Kontrollpflanzen.

Die Resultate sind in der Tabelle 2 zusammengefasst.

Tabelle 2

| Pflanze | Gerste | | | Weizen | | | Mais | | | Lolium perenne | | | Alopecurus myos. | | | Digitaria sanguinalis | | | Echinochloa crus galli | | | Sorghum halepense | | | Rottboellia exaltata | | |
|---|---|---|---|---|---|---|---|---|---|---|---|---|---|---|---|---|---|---|---|---|---|---|---|---|---|---|---|
| Aufwandmenge kg/ha | 4 | 2 | 1 | 4 | 2 | 1 | 4 | 2 | 1 | 4 | 2 | 1 | 4 | 2 | 1 | 4 | 2 | 1 | 4 | 2 | 1 | 4 | 2 | 1 | 4 | 2 | 1 |
| Verbindung | | | | | | | | | | | | | | | | | | | | | | | | | | | |
| 1.006 | 9 | 9 | 9 | 8 | 9 | 9 | 8 | 9 | 9 | 3 | 6 | 9 | 9 | 9 | 9 | 1 | 1 | 1 | 1 | 1 | 2 | 7 | 8 | 9 | 3 | 8 | 9 |
| 1.018 | 9 | 9 | 9 | 9 | 9 | 9 | 9 | 9 | 9 | 2 | 6 | 6 | 9 | 9 | 9 | 1 | 1 | 1 | 1 | 1 | 3 | 5 | 9 | 9 | 3 | 3 | 7 |
| 1.019 | 9 | 9 | 9 | 9 | 9 | 9 | 7 | 9 | 9 | 7 | 8 | 9 | 8 | 9 | 9 | 1 | 1 | 2 | 2 | 2 | 5 | 9 | 9 | 9 | 7 | 9 | 9 |
| 1.029 | 7 | 8 | 9 | 8 | 9 | 9 | 7 | 9 | 9 | 3 | 3 | 5 | 2 | 4 | 6 | 1 | 1 | 1 | 1 | 1 | 4 | 2 | 4 | 6 | 3 | 3 | 6 |
| 1.032 | 9 | 9 | 9 | 9 | 9 | 9 | 7 | 8 | 9 | 7 | 8 | 9 | 3 | 4 | 6 | 1 | 1 | 1 | 1 | 1 | 1 | 9 | 9 | 9 | 4 | 6 | 9 |
| 1.036 | 4 | 5 | 8 | 6 | 8 | 9 | 4 | 7 | 8 | 1 | 1 | 3 | 2 | 2 | 3 | 1 | 1 | 1 | 1 | 1 | 1 | 2 | 2 | 4 | 1 | 2 | 2 |
| 1.037 | 2 | 7 | 9 | 8 | 9 | 9 | 7 | 8 | 9 | 3 | 5 | 8 | 1 | 1 | 3 | 1 | 1 | 1 | 1 | 1 | 3 | 3 | 3 | 5 | 1 | 1 | 2 |
| 1.038 | 6 | 7 | 9 | 7 | 9 | 9 | 7 | 8 | 9 | 1 | 2 | 4 | 1 | 4 | 6 | 1 | 1 | 1 | 1 | 1 | 1 | 1 | 2 | 7 | 1 | 4 | 6 |
| 1.040 | 7 | 9 | 9 | 8 | 9 | 9 | 4 | 5 | 8 | 2 | 2 | 2 | 1 | 2 | 4 | 1 | 1 | 1 | 2 | 3 | 4 | 1 | 2 | 4 | 1 | 1 | 2 |
| 1.042 | 9 | 9 | 9 | 8 | 9 | 9 | 9 | 9 | 9 | 4 | 6 | 7 | 1 | 3 | 8 | 1 | 1 | 1 | 1 | 1 | 2 | 1 | 6 | 9 | 2 | 3 | 4 |
| 1.050 | 9 | 9 | 9 | 9 | 9 | 9 | 9 | 9 | 9 | 2 | 4 | 5 | 7 | 9 | 9 | 1 | 1 | 4 | 1 | 1 | 2 | 9 | 9 | 9 | 7 | 9 | 9 |
| 1.051 | 9 | 9 | 9 | 9 | 9 | 9 | 9 | 9 | 9 | 4 | 4 | 7 | 8 | 9 | 9 | 1 | 2 | 2 | 1 | 1 | 2 | 1 | 3 | 4 | 5 | 6 | 8 |
| 1.058 | 9 | 9 | 9 | 8 | 9 | 9 | 8 | 9 | 9 | 4 | 4 | 6 | 5 | 6 | 6 | 1 | 1 | 2 | 1 | 2 | 4 | 7 | 9 | 9 | 6 | 7 | 9 |
| 1.073 | 9 | 9 | 9 | 9 | 9 | 9 | 6 | 7 | 9 | 5 | 6 | 8 | 1 | 4 | 4 | 1 | 1 | 1 | 1 | 1 | 1 | 2 | 2 | 4 | 1 | 1 | 3 |
| 1.079 | 7 | 8 | 9 | 6 | 7 | 9 | 3 | 4 | 7 | 1 | 1 | 5 | 1 | 2 | 4 | 1 | 1 | 1 | 1 | 1 | 1 | 1 | 1 | 3 | 1 | 1 | 2 |
| 1.080 | 7 | 9 | 9 | 7 | 8 | 9 | 4 | 5 | 7 | 1 | 3 | 5 | 1 | 4 | 8 | 1 | 1 | 1 | 1 | 1 | 4 | 2 | 2 | 6 | 1 | 3 | 3 |

**Die Gräser werden dabei stark, Gerste, Weizen und Mais wenig oder gar nicht geschädigt.**

Beispiel 7: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die geprüften Verbindungen der Tabelle 1 starke bis sehr starke Herbizidwirkung.

Beispiel 8: Herbizidwirkung vor dem Auflaufen der Pflanzen

Kunststofföpfe werden mit expandiertem Vermiculit (Dichte: 0,135 g/cm$^3$, Wasserabsorptionsvermögen: 0,565 l/l) gefüllt. Nach dem Sättigen des nicht adsorptiven Vermiculits mit einer wässrigen Wirkstofemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen ausgewertet.

In diesem Versuch zeigen die geprüften Verbindungen der Tabelle 1 starke Herbizidwirkung.

Beispiel 9: Herbizidwirkung für Wasserreis (paddy)

Selektive Herbizidwirkung in verpflanztem Reis

Reispflänzchen, welche 25 Tage alt sind, werden im Gewächshaus in grosse rechteckige Eternitschalen verpflanzt. Zwischen die Reihen der Reispflanzen werden dann Samen der in Reiskulturen vorkommenden Unkräuter Echinochloa crus galli, Scirpus sp., Monocharia vaginalis und Sagittaria pygmea gesät. Die Schalen werden gut bewässert und bei einer Temperatur von ca. 25°C und hoher Luftfeuchtigkeit gehalten. Nach 12 Tagen, wenn die Unkräuter aufgelaufen sind und das 2-3 Blattstadium erreicht haben, wird die Erde in der Schale mit einer 2,5 cm hohen Schicht Wasser bedeckt. Die Verbindungen werden dann als Emulsionskonzentrate mittels einer Pipette zwischen die Pflanzenreihe appliziert, wobei man das Emulsionskonzentrat so verdünnt und aufträgt, dass es einer Applikationsmenge im Feld von 1 und ½ kg Wirkstoff pro Hektar entspricht. Der Versuch wird nach 4 Wochen ausgewertet. Die Bewertung der Pflanzen geschieht gemäss der obigen Skala.

Die Resultate sind in der Tabelle 3 zusammengefasst.

Tabelle 3

| Pflanze | Reis | | Echinochloa crus galli | | Scirpus | | Monocharia vaginalis | |
|---|---|---|---|---|---|---|---|---|
| Aufwandmenge kg/ha | 1 | 1/2 | 1 | 1/2 | 1 | 1/2 | 1 | 1/2 |
| Verbindung | | | | | | | | |
| 1.001 | 7 | 8 | 1 | 1 | 1 | 1 | 7 | 8 |
| 1.010 | 9 | 9 | 1 | 3 | 3 | 6 | 6 | 6 |
| 1.014 | 8 | 9 | 1 | 1 | 2 | 4 | 7 | 9 |
| 1.018 | 9 | 9 | 2 | 3 | 1 | 1 | 3 | 5 |
| 1.020 | 9 | 9 | 1 | 4 | 5 | 7 | 8 | 9 |
| 1.028 | 9 | 9 | 1 | 1 | 1 | 1 | 3 | 4 |
| 1.029 | 9 | 9 | 1 | 1 | 1 | 1 | 2 | 5 |
| 1.032 | 9 | 9 | 1 | 1 | 1 | 1 | 1 | 1 |
| 1.033 | 9 | 9 | 1 | 2 | 2 | 3 | 7 | 8 |
| 1.035 | 9 | 9 | 1 | 1 | 2 | 5 | 4 | 6 |
| 1.036 | 6 | 6 | 1 | 1 | 1 | 1 | 1 | 1 |
| 1.037 | 6 | 7 | 1 | 1 | 1 | 1 | 1 | 2 |
| 1.038 | 9 | 9 | 1 | 1 | 1 | 1 | 1 | 3 |
| 1.040 | 8 | 9 | 1 | 1 | 1 | 1 | 3 | 5 |
| 1.042 | 8 | 9 | 1 | 1 | 1 | 2 | 3 | 5 |
| 1.051 | 7 | 8 | 1 | 1 | 3 | 5 | 6 | 8 |
| 1.057 | 9 | 9 | 1 | 1 | 1 | 1 | 1 | 4 |
| 1.058 | 8 | 9 | 1 | 1 | 1 | 1 | 3 | 6 |
| 1.073 | 7 | 8 | 1 | 1 | 1 | 1 | 1 | 1 |

Die geprüften Verbindungen schädigen die Unkräuter stark, den Reis nicht oder nur geringfügig.

Beispiel 10: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tab und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit Wirkstoffen der Tabelle 1 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 11: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Temperaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die geprüften Wirkstoffe der Tabelle 1 eine merkliche Erhöhung der Anzahl und des Gewichts der Schoten im Haupttrieb.

Beispiel 12: Wuchshemmung bei Getreide

In Kunststofftöpfen mit sterilisierter Erde wurden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 besprüht. Die Wirkstoffmenge beträgt bis zu 100 g Aktivisubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 13: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabelle 1 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabelle 1 bewirken eine Reduzierung des Neuzuwachses von um 10-30 % im Vergleich zur unbehandelten Kontrolle.

**Patentansprüche**

1. Herbizides und den Pflanzenwuchs regulierendes Mittel, dadurch gekennzeichnet, dass es neben inerten Zutaten als Wirkstoff ein 2-Benzoyl-2-phenyl-oxiran der Formel I enthält.

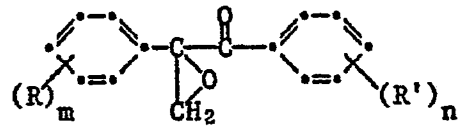

(I)

worin m und n unabhängig voneinander je Null, eins, zwei oder drei R und R′ unabhängig voneinander je ein Halogenatom, eine $C_1$-$C_4$-Alkyl, $C_1$-$C_4$-Alkoxy, $C_1$-$C_4$-Halogenalkyl-, $C_1$-$C_4$-Halogenalkoxy- oder die Nitro- oder Cyangruppe oder einen durch $(R)_m$ substituierten Phenoxyrest bedeutet, als Racemat oder als optisch aktives Enantiomeres.

2. Mittel gemäss Anspruch 1, dadurch gekennzeichnet, dass es als Wirkstoff ein 2-Benzoyl-2-phenyl-oxiran der Formel I, Anspruch 1 enthält, worin m und n unabhängig voneinander je Null, eins, zwei oder drei, und R und R′ unabhängig voneinander je Fluor, Chlor oder Brom, Methyl, Methoxy oder Trifluormethyl bedeuten oder R auch ein durch $(R)_m$ substituierter Phenoxyrest ist, oder ein optisch aktives Enantiomeres einer solchen Substanz.

3. Mittel gemäss Anspruch 1, dadurch gekennzeichnet, dass es als Wirkstoff ein 2-Benzoyl-2-phenyl-oxiran-der Formel I, Anspruch 1 enthält, worin m und n unabhängig voneinander Null, eins, zwei oder drei und R und R′ unabhängig voneinander je Fluor, Chlor, Methyl, Methoxy oder Trifluormethyl bedeutet.

4. Mittel gemäss Anspruch 1, dadurch gekennzeichnet, dass es als Wirkstoff 2-Benzoyl-2-phenyl-oxiran enthält.

5. Mittel gemäss Anspruch 1, dadurch gekennzeichnet, dass es als Wirkstoff 2-(4-Brombenzoyl-2-phenyl)-oxiran enthält.

6. Mittel gemäss Anspruch 1, dadurch gekennzeichnet, dass es als Wirkstoff 2-(4-Chlorbenzoyl)-2-(4-chlorphenyl)oxiran enthält.

7. Mittel gemäss Anspruch 1, dadurch gekennzeichnet, dass es als Wirkstoff 2-(4-Chlorbenzoyl)-2-(2-chlorphenyl)-oxiran enthält.

8. Mittel gemäss Anspruch 1, dadurch gekennzeichnet, dass es als Wirkstoff 2-Benzoyl-2-m-tolyl-oxiran enthält.

9. Mittel gemäss Anspruch 1, dadurch gekennzeichnet, dass es als Wirkstoff 2-(2-Chlorbenzoyl)-2-phenyl-oxiran enthält.

10. Mittel gemäss Anspruch 1, dadurch gekennzeichnet, dass es als Wirkstoff 2-(4-Chlorbenzoyl)-2-(3-chlorphenyl)-oxiran enthält.

11. Mittel gemäss Anspruch 1, dadurch gekennzeichnet, dass es als Wirkstoff 2-(4-Chlorbenzoyl)-2-(3-bromphenyl)-oxiran enthält.

12. Mittel gemäss Anspruch 1, dadurch gekennzeichnet, dass es als Wirkstoff 2-(4-Chlorbenzoyl)-2-phenyl-oxiran enthält.

13. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Mittels gemäss Anspruch 1 behandelt.

14. Verfahren zur selektiven Unkrautbekämpfung in Reiskulturen, dadurch gekennzeichnet, dass man die Kulturen pre- oder postemergent mit einer wirksamen Menge eines Mittels gemäss Anspruch 1 behandelt.

15. Verfahren gemäss Anspruch 14, dadurch gekennzeichnet, dass man den Reis, dessen Anbaufläche oder dessen Samen mit einer wirksamen Menge eines Mittels gemäss Anspruch 1 behandelt.

16. Reissamen, welche mit einer wirksamen Menge eines Mittels gemäss Anspruch 1-7 behandelt worden sind.

17. Verwendung des Mittels gemäss Anspruch 1 zur Kontrolle von Schadgräsern.

18. Die Verwendung des Mittels gemäss Anspruch 1 zur Bekämpfung unerwünschten Pflanzenwachstums.

19. Die Verwendung des Mittels gemäss Anspruch 1 zur Hemmung des Pflanzenwachstums.

20. Die Verwendung des Mittels gemäss Anspruch 1 zur Beeinflussung des Pflanzenwachstums zum Zweck der Ertragssteigerung.

21. Verfahren zur Unterdrückung des Pflanzenwachstums über das 2-Blattstadium hinaus, dadurch gekennzeichnet, dass man die Pflanzen während ihres Wachstums mit einer wirksamen Menge eines Mittels gemäss Anspruch 1 behandelt.

22. Optisch aktive Enantiomere von 2-Benzoyl-2-phenyl-oxiran der Formel I

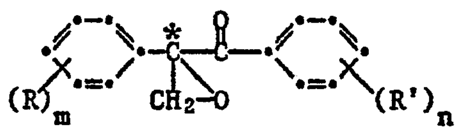

(I*)

worin m und n unabhängig voneinander je null eins, zwei oder drei, R und R′ unabhängig voneinander je ein Halogenatom, eine $C_1$-$C_4$-Alkyl-, $C_1$-$C_4$-Alkoxy-, $C_1$-$C_4$-Halogenalkyl-, $C_1$-$C_4$-Halogenalkoxy- oder die Nitro oder Cyanogruppe oder einen durch $(R)_m$ substituierten Phenoxyrest bedeutet.

23. Neue 2-Benzoyl-2-phenyloxirane der Formel Ia

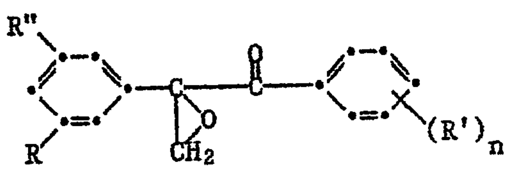

(Ia)

worin R je Halogen, $C_1$–$C_4$-Alkyl oder $C_1$–$C_4$-Alkoxy bedeutet, R″ dasselbe wie R oder Wasserstoff bedeutet und R′ und n die im Anspruch 1 gegebene Bedeutung haben als Racemat oder als optisch aktives Enantiomeres, mit Ausnahme von 2-(3-Chlorbenzoyl)-2-(3-chlorphenyl)-oxiran.

24. Neue 2-Benzoyl-2-phenyloxirane gemäß Anspruch 23 worin R″ Wasserstoff bedeutet.

25. 2-(4-Chlorbenzoyl)-2-(3-methoxyphenyl)-oxiran gemäß Anspruch 23.

## Revendications

1. Produit herbicide et régulateur de la croissance des végétaux, caractérisé en ce qu'il contient en tant que substance active, avec des additifs inertes, un 2-benzoyl-2-phényl-oxiranne de formule I

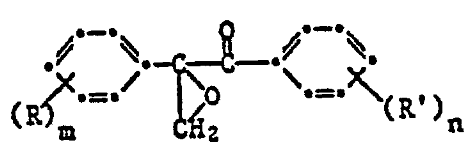

(I)

dans laquelle m et n représentent chacun, indépendamment l'un de l'autre, 0, 1, 2 ou 3, R et R' représentent chacun, indépendamment l'un de l'autre, un atome d'halogène, un groupe alkyle en $C_1$–$C_4$, alcoxy en $C_1$–$C_4$, halogénoalkyle en $C_1$–$C_4$, halogénoalcoxy en $C_1$–$C_4$ ou un groupe nitro ou cyano ou encore un groupe phénoxy substitué par $(R)_m$, à l'état de racémate ou d'énantiomère possédant l'activité optique.

2. Produit selon la revendication 1, caractérisé en ce qu'il contient en tant que substance active un 2-benzoyl-2-phényl-oxiranne de formule I, revendication 1, dans laquelle m et n représentent chacun, indépendamment l'un de l'autre, 0, 1, 2 ou 3 et R et R' représentent chacun, indépendamment l'un de l'autre, le fluor, le chlore ou le brome, un groupe méthyle, méthoxy ou trifluorométhyle, R pouvant également représenter un groupe phénoxy substitué par $(R)_m$, ou un énantiomère, possédant l'activité optique, d'une telle substance.

3. Produit selon la revendication 1, caractérisé en ce qu'il contient en tant que substance active un 2-benzoyl-2-phényl-oxiranne de formule I, revendication 1, dans laquelle m et n représentent chacun, indépendamment l'un de l'autre, 0, 1, 2 ou 3 et R et R' représentent chacun, indépendamment l'un de l'autre, le fluor, le chlore, un groupe méthyle, méthoxy ou trifluorométhyle.

4. Produit selon la revendication 1, caractérisé en ce qu'il contient en tant que substance active le 2-benzoyl-2-phényl-oxiranne.

5. Produit selon la revendication 1, caractérisé en ce qu'il contient en tant que substance active le 2-(4-bromobenzoyl-2-phényl)-oxiranne.

6. Produit selon la revendication 1, caractérisé en ce qu'il contient en tant que substance active le 2-(4-chlorobenzoyl)-2-(4-chlorophényl)-oxiranne.

7. Produit selon la revendication 1, caractérisé en ce qu'il contient en tant que substance active le 2-(4-chlorobenzoyl)-2-(2-chlorophényl)-oxiranne.

8. Produit selon la revendication 1, caractérisé en ce qu'il contient en tant que substance active le 2-benzoyl-2-m-tolyl-oxiranne.

9. Produit selon la revendication 1, caractérisé en ce qu'il contient en tant que substance active le 2-(2-chlorobenzoyl)-2-phényl-oxiranne.

10. Produit selon la revendication 1, caractérisé en ce qu'il contient en tant que substance active le 2-(4-chlorobenzoyl)-2-(3-chlorophényl)-oxiranne.

11. Produit selon la revendication 1, caractérisé en ce qu'il contient en tant que substance active le 2-(4-chlorobenzoyl)-2-(3-bromophényl)-oxiranne.

12. Produit selon la revendication 1, caractérisé en ce qu'il contient en tant que substance active le 2-(4-chlorobenzoyl)-2-phényl-oxiranne.

13. Procédé pour combattre sélectivement en prélevée ou en post-levée les végétaux adventices dans les cultures de végétaux utiles, caractérisé en ce que l'on traite les cultures de végétaux utiles ou leurs aires de plantation par une quantité efficace d'un produit selon la revendication 1.

14. Procédé pour combattre sélectivement les végétaux adventices dans les cultures de riz, caractérisé en ce que l'on traite les cultures en pré-levée ou en post-levée par une quantité efficace d'un produit selon la revendication 1.

15. Procédé selon la revendication 14, caractérisé en ce que l'on traite le riz, son aire de culture ou ses semences par une quantité efficace d'un produit selon la revendication 1.

16. Semences de riz qui ont été traitées par une quantité efficace d'un produit selon les revendications 1 à 7.

17. Utilisation du produit selon la revendication 1 pour la lutte contre les graminées nuisibles.

18. L'utilisation du produit selon la revendication 1 dans la lutte contre les croissances de végétaux indésirables.

19. L'utilisation du produit selon la revendication 1 pour l'inhibition de la croissance des végétaux.

20. L'utilisation du produit selon la revendication 1 pour agir sur la croissance des végétaux dans le sens d'une augmentation de rendement.

21. Procédé pour inhiber la croissance de végétaux au-delà du stade 2 feuilles, caractérisé en ce que l'on traite les végétaux durant leur croissance par une quantité efficace d'un produit selon la revendication 1.

22. Enantiomères, possédant l'activité optique, des 2-benzoyl-2-phényl-oxirannes de formule I

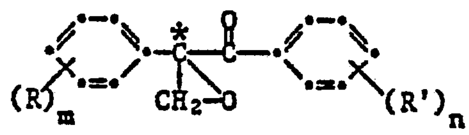

(I*)

dans laquelle m et n représentent chacun, indépendamment l'un de l'autre, 0, 1, 2 ou 3, R et R' représentent chacun, indépendamment l'un de l'autre, un atome d'halogène, un groupe alkyle en $C_1$–$C_4$, alcoxy en $C_1$–$C_4$, halogénoalkyle en $C_1$–$C_4$, halogénoalcoxy en $C_1$–$C_4$, nitro ou cyano, ou un groupe phénoxy substitué par $(R)_m$.

23. Nouveaux 2-benzoyl-2-phényl-oxirannes de formule Ia

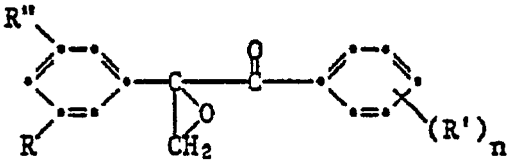

(Ia)

dans laquelle R représente un halogène, un groupe alkyle en $C_1$–$C_4$ ou alcoxy en $C_1$–$C_4$, R″ a les mêmes significations que R ou représente l'hydrogène et R′ et n ont les significations indiquées dans la revendication 1, à l'état de racémates ou d'énantiomères possédant l'activité optique, à l'exception du 2-(3-chlorobenzoyl)-2-(3-chlorophényl)-oxiranne.

24. Nouveaux 2-benzoyl-2-phényl-oxirannes selon la revendication 23, dans lesquels R″ représente l'hydrogène.

25. Le 2-(4-chlorobenzoyl)-2-(3-méthoxyphényl)-oxiranne selon la revendication 23.

**Claims**

1. A herbicidal and plant growth regulating composition which contains as active ingredient a 2-benzoyl-2-phenyl-oxirane of formula I

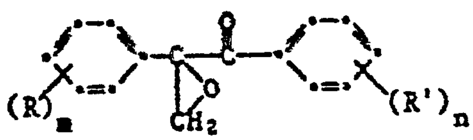

(I)

wherein each of m and n independently of the other is 0, 1, 2 or 3 and each of R and R′ independently of the other is a halogen atom, a $C_1$–$C_4$alkyl, $C_1$–$C_4$alkoxy, $C_1$–$C_4$haloalkyl or $C_1$–$C_4$haloalkoxy group or the nitro or cyano group or a phenoxy group which is substituted by $(R)_m$, in the form of a racemate or an optically active enantiomer, together with inert adjuvants.

2. A composition according to claim 1, which contains as active ingredient a 2-benzoyl-2-phenyloxirane of formula I according to claim 1, wherein each of m and n independently of the other is 0, 1, 2 or 3 and each of R and R′ independently of the other is fluorine, chlorine, bromine, methyl, methoxy or trifluoromethyl, or R is also a phenoxy group which is substituted by $(R)_m$, or an optically active enantiomer of such a substance.

3. A composition according to claim 1, which contains as active ingredient a 2-benzoyl-2-phenyloxirane of formula I according to claim 1, wherein each of m and n independently of the other is 0, 1, 2 or 3 and each of R and R′ independently of the other is fluorine, chlorine, methyl, methoxy or trifluoromethyl.

4. A composition according to claim 1, which contains as active ingredient 2-benzoyl-2-phenyloxirane.

5. A composition according to claim 1, which contains as active ingredient 2-(4-bromobenzoyl-2-phenyl)oxirane.

6. A composition according to claim 1, which contains as active ingredient 2-(4-chlorobenzoyl)-2-(4-chlorophenyl)-oxirane.

7. A composition according to claim 1, which contains as active ingredient 2-(4-chlorobenzoyl)-2-(2-chlorophenyl)-oxirane.

8. A composition according to claim 1, which contains as active ingredient 2-benzoyl-2-m-tolyloxirane.

9. A composition according to claim 1, which contains as active ingredient 2-(2-chlorobenzoyl)-2-phenyloxirane.

10. A composition according to claim 1, which contains as active ingredient 2-(4-chlorobenzoyl)-2-(3-chlorophenyl)-oxirane.

11. A composition according to claim 1, which contains as active ingredient 2-(4-chlorobenzoyl)-2-(3-bromophenyl)-oxirane.

12. A composition according to claim 1, which contains as active ingredient 2-(4-chlorobenzoyl)-2-phenyloxirane.

13. A method of selectively controlling weeds pre- or postemergence in crops of useful plants, which method comprises treating the useful plants or the crop area thereof with an effective amount of a composition according to claim 1.

14. A method of selectively controlling weeds in rice crops, which method comprises treating the crops pre- or postemergence with an effective amount of a composition according to claim 1.

15. A method according to claim 14, which method comprises treating the rice, the crop area thereof or the seeds thereof with an effective amount of a composition according to claim 1.

16. Rice seeds which have been treated with an effective amount of a composition according to claims 1 to 7.

17. Use of a composition according to claim 1 for controlling harmful grasses.

18. Use of a composition according to claim 1 for controlling undesired plant growth.

19. Use of a composition according to claim 1 for inhibiting plant growth.

20. Use of a composition according to claim 1 for influencing plant growth in order to increase yield.

21. A method of inhibiting plant growth beyond the 2-leaf stage, which method comprises treating the plants during their growth with an effective amount of a composition according to claim 1.

22. Optically active enantiomers of 2-benzoyl-2-phenyloxirane of formula I

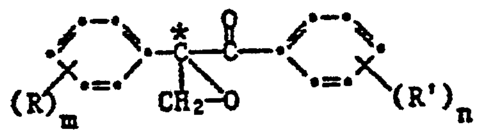

(I*)

wherein each of m and n independently of the other is 0, 1, 2 or 3 and each of R and R′ independently of the other is a halogen atom, a $C_1$–$C_4$alkyl, $C_1$–$C_4$alkoxy, $C_1$–$C_4$haloalkyl or $C_1$–$C_4$haloalkoxy group or the nitro or cyano group or a phenoxy group which is substituted by $(R)_m$.

23. Novel 2-benzoyl-2-phenyloxiranes of formula Ia

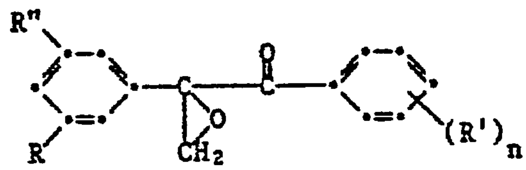

(Ia)

wherein R is halogen, $C_1$–$C_4$alkyl or $C_1$–$C_4$alkoxy, R″ has the same meanings as R or is hydrogen, and R′ and n are as defined in claim 1, in the form of a racemate or an optically active enantiomer, with the exception of 2-(3-chlorobenzoyl)-2-(3-chlorophenyl)oxirane.

24. Novel 2-benzoyl-2-phenyloxiranes according to claim 23, wherein R″ is hydrogen.

25. 2-(4-Chlorobenzoyl)-2-(3-methoxyphenyl)oxirane according to claim 23.